# EUROPÄISCHE PATENTSCHRIFT

(11) **EP 0 696 576 B1**
(45) Veröffentlichungstag und Bekanntmachung des Hinweises auf die Patenterteilung: **12.04.2000**
(21) Anmeldenummer: 95112059.1
(22) Anmeldetag: 01.08.1995
(51) Int. Cl.: C07C 213/02, C07C 217/64, C07C 215/28, C07C 251/48, C07D 213/53, C07D 333/22, C07D 239/26

(54) **Verfahren zur Herstellung von 2-Amino-2-arylethanolen und neue Zwischenprodukte**
Process for the preparation of 2-amino-2-arylethanols and intermediates
Procédé pour la préparation d'amino-2-aryl-2-éthanols et leurs intermédiaires

(30) Priorität: 12.08.1994 DE 4428533
(43) Veröffentlichungstag der Anmeldung: 14.02.1996
(73) Patentinhaber: BAYER AG, 51368 Leverkusen (DE)
(72) Erfinder: Lantzsch, Reinhard, Dr., D-42115 Wuppertal (DE); Kysela, Ernst, Dr., D-51427 Bergisch Gladbach (DE); Marhold, Albrecht, Dr., D-51373 Leverkusen (DE)

(56) Entgegenhaltungen:
- EP-A- 0 311 385
- JOURNAL OF ORGANIC CHEMISTRY, Bd. 48, Nr. 15, 1983, EASTON US, Seiten 2520-2527, XP002022795 WILLIAM H. PIRKLE ET AL.: "Improved Chiral Derivatizing Agents..."
- JOURNAL OF ORGANIC CHEMISTRY, Bd. 46, Nr. 4, 1981, EASTON US, Seiten 742-750, XP002022796 ANGELO ALBERTI ET AL.: "Stereochemistry of Iminoxy Radicals"

## Beschreibung

Die Erfindung betrifft ein neues Verfahren zur Herstellung von neuen 2-Amino-2-arylethanolen und neue Zwischenprodukte zu ihrer Herstellung.

Es ist bekannt, daß man 2-Amino-2-aryl-ethanole und ihre Derivate erhält, wenn man entsprechend substituierte aromatische Aminosäuren oder ihre Ester oder Oximether oder Hydroxyoxime mit komplexen Metallhydriden wie beispielsweise NaBH₄/BF₃ oder LiAlH₄ reduziert (vgl. z.B. EP 322982, Z. Anorg. Allg. Chemie 1970, 376, 296-302 oder Comprehensive Organic Synthesis, Band 8, S. 64ff.). Die Verwendung dieser Metallhydride ist großtechnisch allerdings nur in beschränktem Umfang möglich, da sie einerseits sehr teuer sind und andererseits die Umsetzungen einen erheblichen Sicherheitsaufwand erfordern.

Weiter ist bekannt, daß bestimmte Amine durch katalytische Hydrierung mit Edelmetall-Katalysatoren, wie beispielsweise Rhodium- oder Palladiumkatalysatoren und gegebenenfalls in Gegenwart von Säuren, wie beispielsweise Essigsäure, Schwefelsäure oder Chlorwasserstoff erhalten werden können (vgl. z.B. J. Org. Chem. 27, 2209, 1962; J. Org. Chem. 28, 2797, 1963; J. Org. Chem. 48, 2520 (1983). Diese Verfahren weisen jedoch den Nachteil auf, daß sie nicht ohne Einschränkungen anwendbar sind. So gelingt beispielsweise mit Rhodium als Katalysator die Reduktion des Indanon-2-oxims zu dem entsprechenden Amin nicht (vgl. Houben-Weyl, Methoden der Organischen Chemie, Georg Thieme Verlag, Stuttgart, Band IV, lc, Seite 251-252). Auch Palladium-Katalysatoren können nicht universell eingesetzt werden, da zum Beispiel aus halogensubstituierten Aromaten das Halogen reduktiv entfernt wird.

Gegenstand der vorliegenden Erfindung ist
(1) ein Verfahren zur Herstellung von 2-Amino-2-aryl-ethanolen der Formel (I) 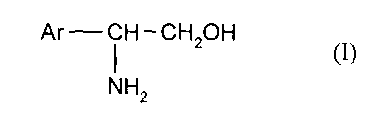 in welcher
   - Ar: für jeweils einfach bis fünffach, gleich oder verschieden substituiertes Phenyl oder jeweils einfach bis dreifach, gleich oder verschieden substituiertes Thienyl, Pyridyl oder Pyrimidinyl steht, wobei jeweils als Substituenten genannt seien:
   Fluor, Chlor, Brom, C₁-C₆-Alkyl, C₁-C₆-Halogenalkyl, C₁-C₆-Alkoxy, C₁-C₆-Halogenalkoxy, gegebenenfalls durch Halogen oder C₁-C₆-Alkyl substituiertes C₃-C₆-Cycloalkyloxy und gegebenenfalls einfach bis fünffach, gleich oder verschieden durch Fluor, Chlor, Brom, C₁-C₆-Alkyl, C₁-C₆-Halogenalkyl, C₁-C₆-Alkoxy, C₁-C₆-Halogenalkoxy oder gegebenenfalls durch Halogen oder C₁-C₆-Alkyl substituiertes C₃-C₆-Cycloalkyloxy substituiertes Phenyl,
   dadurch gekennzeichnet, daß man α-Hydroxyketoxime der Formel (II) 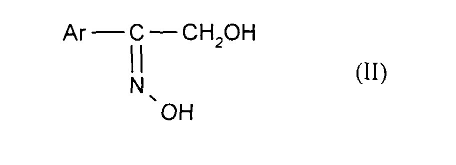 in welcher
   - Ar: die oben angegebene Bedeutung hat,
   mit Raney-Nickel oder Raney-Cobalt in Gegenwart eines Verdünnungsmittels und in Gegenwart einer Base katalytisch hydriert;
(2) neue 2-Amino-2-arylethanole der Formel (Ia) 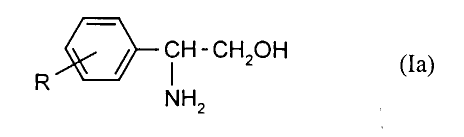 in welcher
   - R: für einen Rest 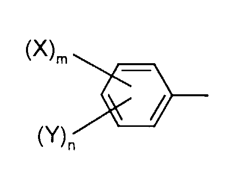 steht,
   in welchem
   - X: für Halogen, C₁-C₆ Alkyl oder C₁-C₆ Alkoxy steht,
   - Y: für C₁-C₆ Halogenalkoxy oder durch Halogen substituiertes C₃-C₆ Cycloalkyloxy steht,
   - m: für die Zahlen 0, 1 oder 2 steht und
   - n: für die Zahlen 1 oder 2 steht;
(3) neue α-Hydroxy-ketoxime der Formel (IIa) 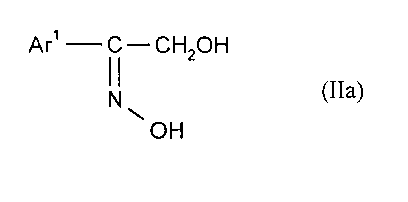 in welcher
   - Ar¹: für einfach bis fünffach, gleich oder verschieden substituiertes Phenyl, oder jeweils einfach bis dreifach, gleich oder verschieden substituiertes Thienyl, Pyridyl oder Pyrimidinyl steht, wobei als Substituenten genannt seien:
   Fluor, Chlor, Brom, C₂-C₆-Alkoxy, C₁-C₆-Halogenalkoxy, gegebenenfalls durch Halogen oder C₁-C₆-Alkyl substituiertes C₃-C₆-Cycloalkyloxy und einfach bis fünffach, gleich oder verschieden durch Fluor, Chlor, Brom, C₁-C₆-Alkyl, C₁-C₆-Alkoxy, C₁-C₆-Halogenalkoxy oder gegebenenfalls durch Halogen oder C₁-C₆-Alkyl substituiertes C₃-C₆-Cycloalkyloxy substituiertes Phenyl,
   ausgenommen die Verbindung 1-(5-Brom-2-thienyl)-2-hydroxy-ethanonoxim und ausgenommen Verbindungen, in welcher Ar¹ eine der folgenden Bedeutungen annimmt:
   4-Chlorphenyl, 2-Chlorphenyl, 2-Bromphenyl, 2,6-Dichlorphenyl, 2-Chlor-6-fluorphenyl, Pentafluorphenyl.

Es ist als ausgesprochen überraschend anzusehen, daß nach dem erfindungsgemäßen Verfahren (1) die 2-Amino-2-arylethanole der Formel (I) in sehr guten Ausbeuten und in hoher Reinheit erhalten werden, da bekannt ist, daß bei der katalytischen Hydrierung von Ketoximen und Verwendung von Platin- oder Raney-Nickel-Katalysatoren Hydroxylamine entstehen [Houben-Weyl, Methoden der Organischen Chemie, Georg Thieme Verlag, Stuttgart, Band IV, 1c, Teil 1, Seite 251; JACS 59, 715 (1937) und Chem. Ber. 88, 38 (1955)]. Das erfindungsgemäße Verfahren zur Herstellung der oben unter (1) bezeichneten 2-Amino-2-arylethanole der Formel (I) ist hingegen breit anwendbar und stellt somit eine wertvolle Bereicherung der Technik dar.

Nach dem erfindungsgemäßen Verfahren werden bevorzugt Verbindungen der Formel (I) hergestellt, in welcher
- Ar: für jeweils einfach bis dreifach, gleich oder verschieden substituiertes Phenyl, Thienyl, Pyridyl oder Pyrimidinyl steht, wobei als Substituenten genannt seien:
Fluor, Chlor, Brom, C₁-C₄-Alkyl, C₁-C₄-Halogenalkyl, C₁-C₄-Alkoxy, C₁-C₄-Halogenalkoxy, gegebenenfalls durch Halogen substituiertes C₃-C₆-Cycloalkyloxy und gegebenenfalls einfach bis dreifach, gleich oder verschieden durch Fluor, Chlor, Brom, C₁-C₄-Alkyl, C₁-C₄-Halogenalkyl, C₁-C₄-Alkoxy, C₁-C₄-Halogenalkoxy oder gegebenenfalls durch Halogen substituiertes C₃-C₆-Cycloalkyloxy substituiertes Phenyl.

Nach dem erfindungsgemäßen Verfahren werden besonders bevorzugt Verbindungen der Formel (I) hergestellt, in welcher
- Ar: für einfach oder zweifach, gleich oder verschieden substituiertes Phenyl steht, wobei als Substituenten genannt seien: Fluor, Chlor, Brom, gegebenenfalls durch Fluor und/oder Chlor substituiertes C₁-C₄-Alkyl, gegebenenfalls durch Fluor und/oder Chlor substituiertes C₁-C₄-Alkoxy, gegebenenfalls einfach oder mehrfach durch Fluor und/oder Chlor substituiertes C₃-C₆-Cycloalkyloxy und gegebenenfalls einfach oder zweifach, gleich oder verschieden durch Fluor, Chlor, Brom, gegebenenfalls durch Fluor und/oder Chlor substituiertes C₁-C₄-Alkyl, gegebenenfalls durch Fluor und/oder Chlor substituiertes C₁-C₄-Alkoxy oder gegebenenfalls einfach oder mehrfach durch Fluor und/oder Chlor substituiertes C₃-C₆-Cycloalkyloxy substituiertes Phenyl.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Diese Restedefinitionen können untereinander, also auch zwischen den jeweiligen Bereichen und Vorzugsbereichen beliebig kombiniert werden.

Verwendet man beispielsweise als Ausgangsstoff das Oxim des 4-Difluormethoxy-ω-hydroxy-acetophenons, Wasserstoff und als Katalysator Raney-Nickel, so läßt sich der Reaktionsablauf des erfindungsgemäßen Verfahrens durch das folgende Formelschema darstellen:

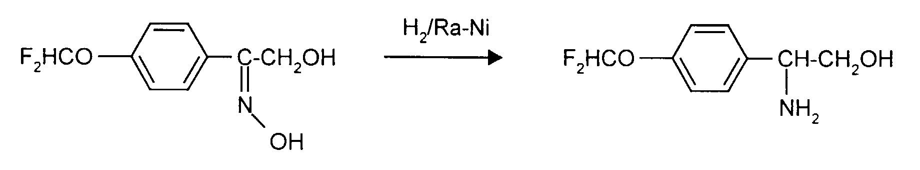

Die bei dem unter (1) angegebenen erfindungsgemäßen Verfahren zur Herstellung der Verbindungen der Formel (I) als Ausgangsstoffe zu verwendenden -Hydroxy-ketoxime sind durch die Formel (II) allgemein definiert. In der Formel (II) steht Ar bevorzugt bzw. besonders bevorzugt für diejenigen Bedeutungen, die bereits oben im Zusammenhang mit der Beschreibung der Verbindungen der Formel (I) als bevorzugt bzw. besonders bevorzugt für Ar angegeben wurden.

Die α-Hydroxy-ketoxime der Formel (II) sind teilweise bekannt (vgl. z.B. J. Chem. Technol. Biotechnol., 54(1), 19-26, 1992).

Noch nicht bekannt und ebenfalls Gegenstand dieser Erfindung sind jedoch die oben unter (3) angegebenen und durch die Formel (IIa) definierten α-Hydroxyketoxime.
- Ar¹: steht bevorzugt für jeweils einfach bis dreifach, gleich oder verschieden substituiertes Phenyl, Thienyl, Pyridyl oder Pyrimidinyl, wobei als Substituenten genannt seien:
Fluor, Chlor, Brom, C₂-C₄-Alkoxy, C₁-C₄-Halogenalkoxy, gegebenenfalls durch Halogen substituiertes C₃-C₆-Cycloalkyloxy und einfach bis dreifach, gleich oder verschieden durch Fluor, Chlor, Brom, C₁-C₄-Alkyl, C₁-C₄-Alkoxy, C₁-C₄-Halogenalkoxy oder gegebenenfalls durch Halogen substituiertes C₃-C₆-Cycloalkyloxy substituiertes Phenyl, ausgenommen die Verbindung 1-(5-Brom-2-thienyl)-2-hydroxy-ethanonoxim und ausgenommen Verbindungen, in welcher Ar' eine der folgenden Bedeutungen annimmt: 4-Chlorphenyl, 2-Chlorphenyl, 2-Bromphenyl, 2,6-Dichlorphenyl, 2-Chlor-6-fluorphenyl, Pentafluorphenyl.
- Ar¹: steht ganz besonders bevorzugt für einfach oder zweifach, gleich oder verschieden substituiertes Phenyl, wobei als Substituenten genannt seien:
Fluor, Chlor, Brom, C₂-C₄-Alkoxy, durch Fluor und/oder Chlor substituiertes C₁-C₄-Alkoxy, gegebenenfalls einfach oder mehrfach durch Fluor und/oder Chlor substituiertes C₃-C₆-Cycloalkyloxy und einfach oder zweifach, gleich oder verschieden durch Fluor, Chlor, Brom, C₁-C₄-Alkyl, gegebenenfalls durch Fluor und/oder Chlor substituiertes C₁-C₄-Alkoxy, oder gegebenenfalls einfach oder mehrfach durch Fluor und/oder Chlor substituiertes C₃-C₆-Cycloalkyloxy substituiertes Phenyl, ausgenommen die Verbindung 1-(5-Brom-2-thienyl)-2-hydroxy-ethanonoxim und ausgenommen Verbindungen, in welcher Ar¹ eine der folgenden Bedeutungen annimmt:
4-Chlorphenyl, 2-Chlorphenyl, 2-Bromphenyl, 2,6-Dichlorphenyl, 2-Chlor-6-fluorphenyl, Pentafluorphenyl.

Dabei sind die Verbindungen 1-(4-Methoxyphenyl)-2-hydroxy-ethanon-oxim und 1-(5-Brom-2-thienyl)-2-hydroxy-ethanonoxim (Khimiya i Tekhnol, Elementoorg. Poluproduktov i Polimerov, Volgograd (1984) 20-4, from: Ref. Zh., Khim, 1985, Abstr. No. 9Zh241; Huaxue Xuebao (1983), 41(4), 380-4, CODEN: HHHPA4; ISSN: 0567-7351) jeweils ausgenommen.

Die bekannten und auch die neuen Verbindungen der Formel (II) können nach allgemein bekannten Verfahren und auf übliche Weise aus den entsprechenden ω-Hydroxyketonen und Hydroxylaminhydrochlorid erhalten werden (vgl. Herstellungsbeispiel).

Die ω-Hydroxyketone erhält man z.B. durch Umsetzung der entsprechenden ω-Chlorketone, die ihrerseits beispielsweise durch Friedel-Crafts-Acylierung substituierter Benzole mit Chloracetylchlorid in Gegenwart von Aluminiumchlorid zugänglich sind, mit Salzen der Ameisensäure (vgl. die Herstellungsbeispiele).

Das unter (1) beschriebene Verfahren wird in Gegenwart von Raney-Nickel oder Raney-Cobalt durchgeführt. Dabei handelt es sich um wohlbekannte Katalysatoren, die z.B. in Houben-Weyl, Methoden der Organischen Chemie, Georg Thieme Verlag, Stuttgart, Band 4/lc (Reduktionen) S. 18 ff., beschrieben sind.

Das unter (1) beschriebene erfindungsgemäße Verfahren wird vorzugsweise in Gegenwart eines Verdünnungsmittels durchgeführt.

Als Verdünnungsmittel kommen alle unter den Reaktionsbedingungen inerten, üblichen organischen Lösungsmittel in Betracht. Hierzu gehören Alkohole, wie beispielsweise Methanol, Ethanol, n-Propanol, iso-Propanol, n-Butanol oder iso-Butanol; Kohlenwasserstoffe, wie beispielsweise Benzol, Toluol, Xylol, Hexan, Cyclohexan oder Ether, wie beispielsweise Diethylether, Diisopropylether, tert. Butylmethylether, tert. Amylmethylether, 1,2-Dimethoxyethan, Tetrahydrofuran und Dioxan.

Unter den Reaktionsbedingungen kann es jedoch auch vorteilhaft sein, Gemische aus Wasser und einem der oben angegebenen Verdünnungsmittel einzusetzen.

Die Reaktionstemperaturen können bei der Durchführung des unter (1) beschriebenen erfindungsgemäßen Verfahrens in einem größeren Bereich variiert werden. Im allgemeinen arbeitet man bei Temperaturen zwischen 15°C und 180°C, vorzugsweise bei Temperaturen zwischen 30°C und 120°C.

Das unter (1) beschriebene erfindungsgemäße Verfahren wird in Gegenwart von Wasserstoff und im allgemeinen unter Druck durchgeführt, wobei der Wasserstoffdruck zwischen einem und 150 bar, vorzugsweise zwischen 15 und 100 bar liegt.

Das unter (1) beschriebene erfindungsgemäße Verfahren wird unter basischen Reaktionsbedingungen, vorzugsweise in Gegenwart von Ammoniak durchgeführt. Dieser kann gegebenenfalls gasförmig oder flüssig eingesetzt werden.

Auf beispielsweise 1 Mol α-Hydroxyketoxim der Formel (II) werden 1 bis 100 Äquivalente, vorzugsweise 5 bis 50 Äquivalente Ammoniak und 1 bis 150 Gewichtsprozent Raney-Nickel oder Raney-Cobalt verwendet, vorzugsweise 10 bis 60 Gewichtsprozent.

Die Aufarbeitung kann nach üblichen Methoden erfolgen, beispielsweise durch Abfiltrieren des Katalysators und Entfernen des Lösungsmittels unter vermindertem Druck. Das so erhaltene Rohprodukt kann durch Umkristallisation oder Chromatographie gereinigt werden. Die Aminoalkohole können auch durch Bildung ihrer Salze, z.B. der Hydrochloride gereinigt werden.

Die oben unter (2) angegebenen 2-Amino-2-arylethanole sind durch die Formel (Ia) allgemein definiert. Für diese Formel gilt, daß für m = 2 bzw. n = 2 die Reste X bzw. Y gleich oder verschieden sein können.

In dem Rest R der Formel (Ia) steht
- X: bevorzugt für Fluor, Chlor, Brom, C₁-C₄-Alkyl oder C₁-C₄-Alkoxy,
- Y: bevorzugt für C₁-C₄-Halogenalkoxy oder für durch Halogen substituiertes C₃-C₆-Cycloalkoxy,
- n: bevorzugt für die Zahl 1 und
- m: bevorzugt für 0 oder 1.

Besonders bevorzugt sind Verbindungen der Formel (Ia) in welcher
- R: für einen Rest 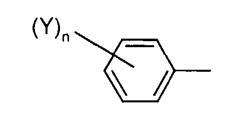 steht,
in welchem
- Y: für durch Fluor und/oder Chlor substituiertes C₁-C₄-Alkoxy oder für durch Fluor und/oder Chlor substituiertes C₃-C₆-Cycloalkoxy und
- n: für die Zahl 1 steht.

Ganz besonders bevorzugt sind Verbindungen der Formel (Ia) in welcher
- R: für einen Rest 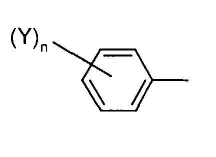 steht,
in welchem
- Y: für einen der folgenden Reste:
-OCHF₂, -OCClF₂, -OCF₂CHFCl, -OCF₂CH₂F, -OCF₂CHF₂, -OCF₂CCl₃, -OCF₂CHFCF₃, -OCH₂CF₃, -OCH₂CF₂CHF₂, -OCH₂CF₂CF₃, 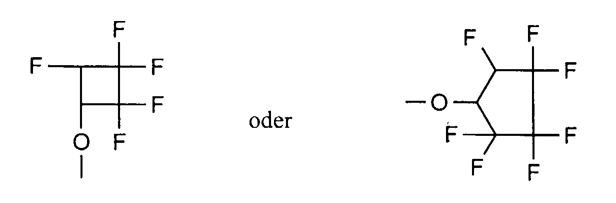
steht und
- n: für eine Zahl 1 steht.

Die 2-Amino-2-arylethanole der Formel (Ia) sind neu und Gegenstand der Erfindung. Sie können nach dem erfindungsgemäßen Verfahren (1) erhalten werden.

Die nach dem erfindungsgemäßen Verfahren herzustellenden 2-Amino-2-arylethanole der Formel (1) können als Ausgangsstoffe zur Herstellung von Schädlingsbekämpfungsmitteln verwendet werden (vgl. z.B. EP 432661).

### Herstellungsbeispiele

### Beispiel 1

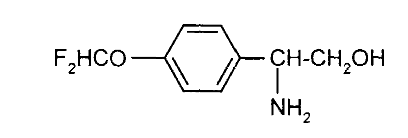

9,8 g (0,045 Mol) 2-Hydroxy-1-(4-difluormethoxy-phenyl)-ethanon-oxim in 100 ml Methanol, 25 ml flüssigem Ammoniak und 5 g Raney-Nickel werden bei 50°C und einem Wasserstoffdruck von 60 bis 70 bar hydriert, bis kein Wasserstoff mehr aufgenommen wird. Nach dem Abkühlen wird entspannt, abfiltriert und das Lösungsmittel unter vermindertem Druck entfernt. Zurück bleibt ein dunkles Öl, das langsam kristallisiert.

Man erhält 7,8 g (84,5% der Theorie) 2-Amino-2-(4-difluormethoxyphenyl)-ethanol mit einer Reinheit von >99% (gaschromatographisch) und einem Schmelzpunkt von 54-57°C.

Auf analoge Weise und gemäß den allgemeinen Angaben zur Herstellung werden die nachfolgend in Tabelle 1 aufgeführten Verbindungen der Formel (I) erhalten:

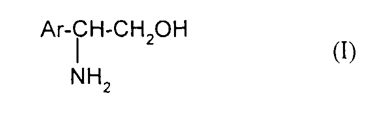

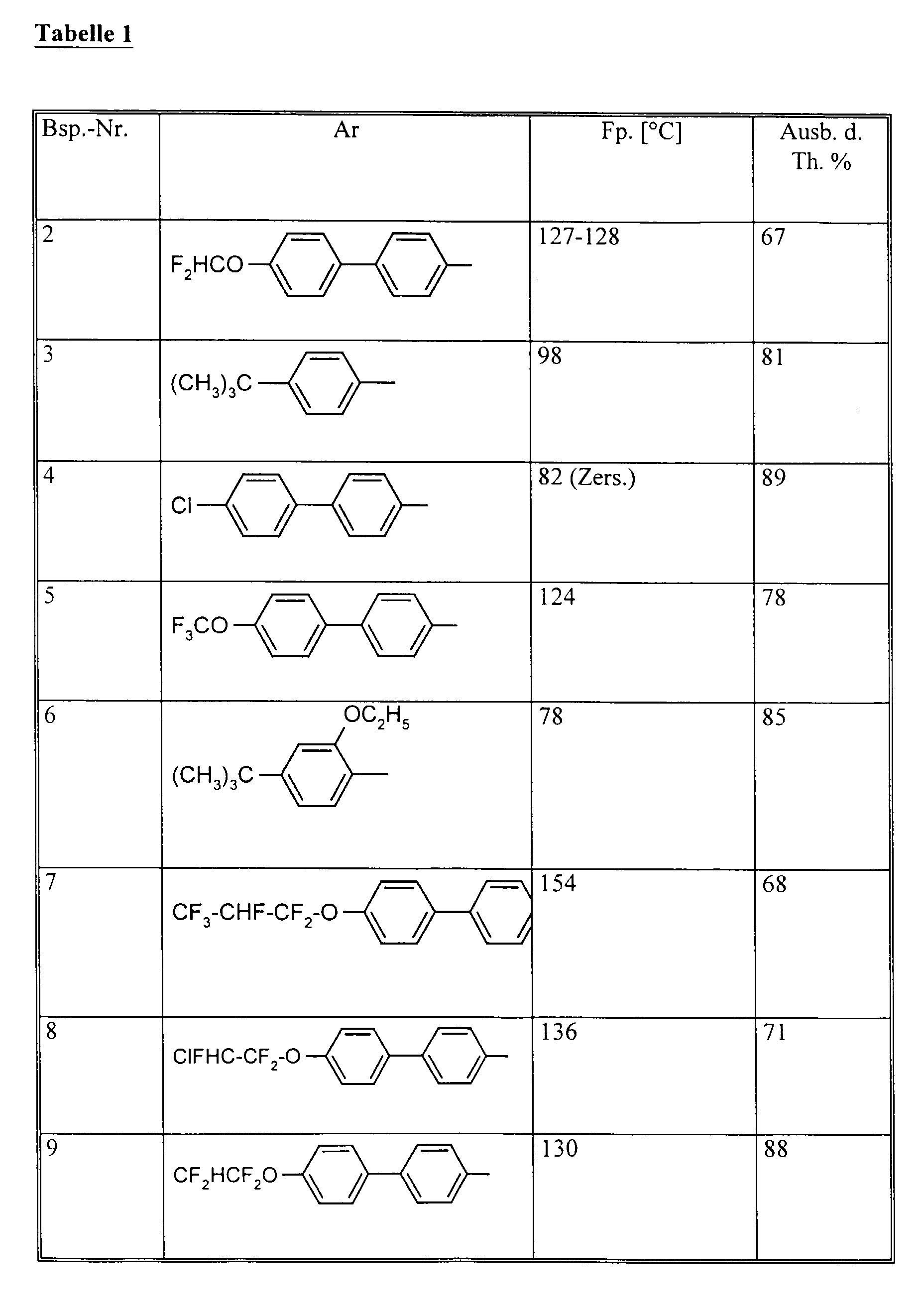

### Herstellung der Ausgangsstoffe:

### Beispiel (II-1)

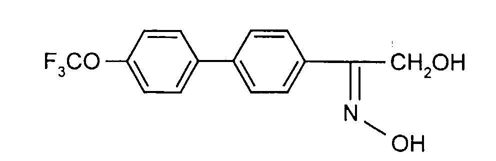

5,9 g (0,02 Mol) 4-Trifluormethoxyphenyl- -hydroxyacetophenon werden in 50 ml Dimethoxyethan vorgelegt, 5 ml Wasser zugegeben, danach 2,1 g (0,025 Mol) Natriumacetat, sowie 1,7 g (0,025 Mol) Hydroxylaminhydrochlorid und 12 Stunden bei Raumtemperatur gerührt. Man gießt auf Eiswasser und filtriert ab. Ausbeute: 5,9 g (88% der Theorie) Schmelzpunkt 150-157°C (E/Z: 25:75)

Analog zu Beispiel II-1 und gemäß den allgemeinen Angaben zur Herstellung werden die nachfolgend in Tabelle 2 aufgeführten Verbindungen der Formel (II) erhalten:

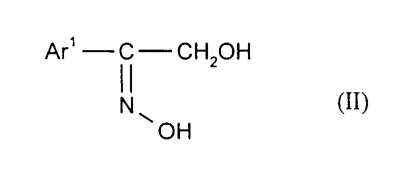

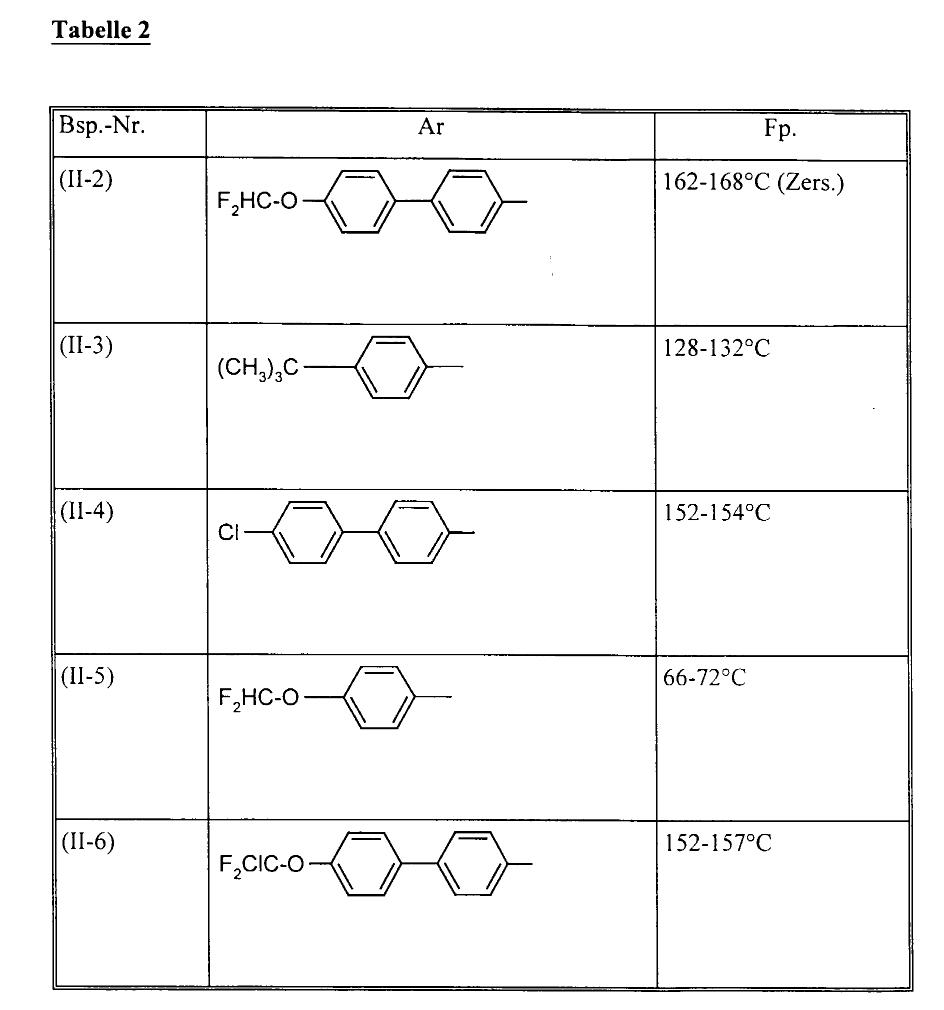

### Beispiel für die Herstellung eines -Hydroxyacetophenons

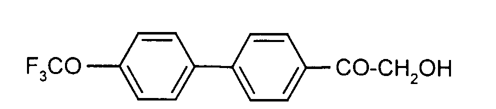

6,75 g (0,0215 Mol) 4-Trifluormethoxyphenyl- -chloracetophenon werden in Ethanol/Wasser suspendiert und 9,2 g (0,1358 Mol) Natriumformiat hinzugefügt. Man erhitzt 12 Stunden zum Sieden, destilliert das Ethanol weitgehend ab, verdünnt mit Wasser und filtriert ab.

Man erhält 6,5 g beige Kristalle (90% der Theorie) vom Schmelzpunkt 165°C.

### Beispiel für die Herstellung eines ω-Chloracetophenons

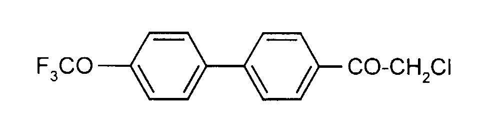

7 g (0,0525 Mol) Aluminiumchlorid wird in 50 ml 1,2-Dichlorethan vorgelegt. Bei 5 bis 10°C werden 5,9 g (0,0525 Mol) Chloracetylchlorid zugetropft und anschließend 11,9 g (0,05 Mol) 4-Trifluormethoxybiphenyl bei -10°C portionsweise zugegeben. Es wird 12 Stunden bei Raumtemperatur nachgerührt, das Reaktionsgemisch auf Eiswasser gegossen und mit Essigsäureethylester extrahiert. Die organische Phase wird abgetrennt, einmal mit Natriumbicarbonatlösung und zweimal mit Wasser gewaschen, getrocknet und eingeengt.

Man erhält 14,3 g Rohprodukt, das über eine Kieselgelsäule (Laufmittel: Methylenchlorid) gereinigt wird. Man erhält 6,85 g hellbeige Kristalle vom Schmelzpunkt 74-76°C. Ausbeute: 38,5% der Theorie.

## Patentansprüche

1. Verfahren zur Herstellung von Verbindungen der Formel (I) 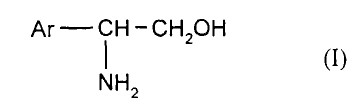 in welcher
Ar für jeweils einfach bis fünffach, gleich oder verschieden substituiertes Phenyl oder jeweils einfach bis dreifach, gleich oder verschieden substituiertes Thienyl, Pyridyl oder Pyrimidinyl steht, wobei jeweils als Substituenten genannt seien:
Fluor, Chlor, Brom, C₁-C₆-Alkyl, C₁-C₆-Halogenalkyl, C₁-C₆-Alkoxy, C₁-C₆-Halogenalkoxy, gegebenenfalls durch Halogen oder C₁-C₆-Alkyl substituiertes C₃-C₆-Cycloalkyloxy und gegebenenfalls einfach bis fünffach, gleich oder verschieden durch Fluor, Chlor, Brom, C₁-C₆-Alkyl, C₁-C₆-Halogenalkyl, C₁-C₆-Alkoxy, C₁-C₆-Halogenalkoxy oder gegebenenfalls durch Halogen oder C₁-C₆-Alkyl substituiertes C₃-C₆-Cycloalkyloxy substituiertes Phenyl,
dadurch gekennzeichnet, daß man Verbindungen der Formel (II) 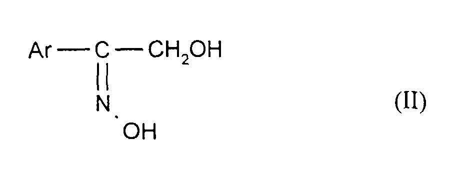 in welcher
Ar die oben angegebene Bedeutung hat,
mit Raney-Nickel oder Raney-Cobalt in Gegenwart eines Verdünnungsmittels und in Gegenwart einer Base katalytisch hydriert.

2. Verfahren zur Herstellung von Verbindungen der Formel (I) gemäß Anspruch 1, in welcher
Ar für jeweils einfach bis dreifach, gleich oder verschieden substituiertes Phenyl, Thienyl, Pyridyl oder Pyrimidinyl steht, wobei als Substituenten genannt seien:
Fluor, Chlor, Brom, C₁-C₄-Alkyl, C₁-C₄-Halogenalkyl, C₁-C₄-Alkoxy, C₁-C₄-Halogenalkoxy, gegebenenfalls durch Halogen substituiertes C₃-C₆-Cycloalkyloxy und gegebenenfalls einfach bis dreifach, gleich oder verschieden durch Fluor, Chlor, Brom, C₁-C₄-Alkyl, C₁-C₄-Halogenalkyl, C₁-C₄-Alkoxy, C₁-C₄-Halogenalkoxy oder gegebenenfalls durch Halogen substituiertes C₃-C₆-Cycloalkyloxy substituiertes Phenyl.

3. Verfahren zur Herstellung von Verbindungen der Formel (I) gemäß Anspruch 1, in welcher
Ar für einfach oder zweifach, gleich oder verschieden substituiertes Phenyl steht, wobei als Substituenten genannt seien: Fluor, Chlor, Brom, gegebenenfalls durch Fluor und/oder Chlor substituiertes C₁-C₄-Alkyl, gegebenenfalls durch Fluor und/oder Chlor substituiertes C₁-C₄-Alkoxy, gegebenenfalls einfach oder mehrfach durch Fluor und/oder Chlor substituiertes C₃-C₆-Cycloalkyloxy und gegebenenfalls einfach oder zweifach, gleich oder verschieden durch Fluor, Chlor, Brom, gegebenenfalls durch Fluor und/oder Chlor substituiertes C₁-C₄-Alkyl, gegebenenfalls durch Fluor und/oder Chlor substituiertes C₁-C₄-Alkoxy oder gegebenenfalls einfach oder mehrfach durch Fluor und/oder Chlor substituiertes C₃-C₆-Cycloalkyloxy substituiertes Phenyl.

4. Verbindungen der Formel (Ia) 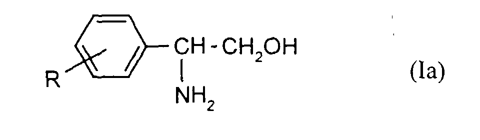 in welcher
R für einen Rest 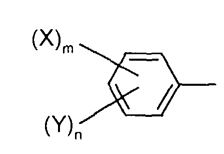 steht,
in welchem
X für Halogen, C₁-C₆ Alkyl oder C₁-C₆ Alkoxy steht,
Y für C₁-C₆ Halogenalkoxy oder durch Halogen substituiertes C₃-C₆ Cycloalkyloxy steht,
m für die Zahlen 0, 1 und 2 steht und
n für die Zahlen 1 und 2 steht.

5. Verbindungen der Formel (Ia) gemäß Anspruch 4, in welcher
X für Fluor, Chlor, Brom, C₁-C₄-Alkyl oder C₁-C₄-Alkoxy,
Y für C₁-C₄-Halogenalkoxy oder für durch Halogen substituiertes C₃-C₆-Cycloalkoxy,
n für die Zahl 1 und
m für 0 oder 1 steht.

6. Verbindungen der Formel (Ia) gemäß Anspruch 4, in welcher
R für einen Rest 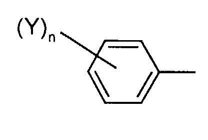 steht,
in welchem
Y für durch Fluor und/oder Chlor substituiertes C₁-C₄-Alkoxy oder für durch Fluor und/oder Chlor substituiertes C₃-C₆-Cycloalkoxy und
n für die Zahl 1 steht.

7. Verbindungen der Formel (Ia) gemäß Anspruch 4, in welcher
R für einen Rest 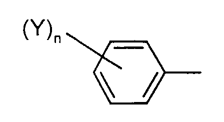 steht,
in welchem
Y für einen der folgenden Reste:
-OCHF₂, -OCClF₂, -OCF₂CHFCl, -OCF₂CH₂F, -OCF₂CHF₂, -OCF₂CCl₃, -OCF₂CHFCF₃, -OCH₂CF₃, -OCH₂CF₂CHF₂, -OCH₂CF₂CF₃, 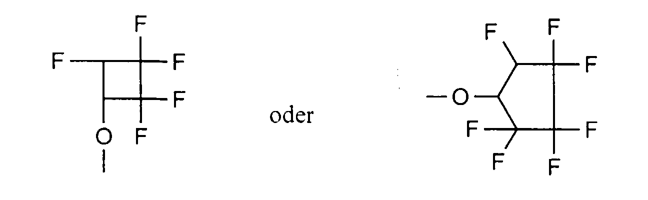
steht und
n für die Zahl 1 steht.

8. Verbindungen der Formel (IIa) 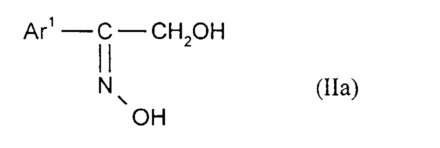 in welcher
Ar¹ für einfach bis fünffach, gleich oder verschieden substituiertes Phenyl, oder jeweils einfach bis dreifach, gleich oder verschieden substituiertes Thienyl, Pyridyl oder Pyrimidinyl steht, wobei als Substituenten genannt seien:
Fluor, Chlor, Brom, C₂-C₆-Alkoxy, C₁-C₆-Halogenalkoxy, gegebenenfalls durch Halogen oder C₁-C₆-Alkyl substituiertes C₃-C₆-Cycloalkyloxy und einfach bis fünffach, gleich oder verschieden durch Fluor, Chlor, Brom, C₁-C₆-Alkyl, C₁-C₆-Alkoxy, C₁-C₆-Halogenalkoxy oder gegebenenfalls durch Halogen oder C₁-C₆-Alkyl substituiertes C₃-C₆-Cycloalkyloxy substituiertes Phenyl,
ausgenommen die Verbindung 1-(5-Brom-2-thienyl)-2-hydroxy-ethanonoxim und ausgenommen Verbindungen, in welcher Ar¹ eine der folgenden Bedeutungen annimmt:
4-Chlorphenyl, 2-Chlorphenyl, 2-Bromphenyl, 2,6-Dichlorphenyl, 2-Chlor-6-fluorphenyl, Pentafluorphenyl.

9. Verbindungen der Formel (IIa) gemäß Anspruch 8, in welcher
Ar¹ für jeweils einfach bis dreifach, gleich oder verschieden substituiertes Phenyl, Thienyl, Pyridyl oder Pyrimidinyl steht, wobei als Substituenten genannt seien:
Fluor, Chlor, Brom, C₂-C₄-Alkoxy, C₁-C₄-Halogenalkoxy, gegebenenfalls durch Halogen substituiertes C₃-C₆-Cycloalkyloxy und einfach bis dreifach, gleich oder verschieden durch Fluor, Chlor, Brom, C₁-C₄-Alkyl, C₁-C₄-Alkoxy, C₁-C₄-Halogenalkoxy oder gegebenenfalls durch Halogen substituiertes C₃-C₆-Cycloalkyloxy substituiertes Phenyl,
ausgenommen die Verbindung 1-(5-Brom-2-thienyl)-2-hydroxyethanonoxim und ausgenommen Verbindungen, in welcher Ar¹ eine der folgenden Bedeutungen annimmt:
4-Chlorphenyl, 2-Chlorphenyl, 2-Bromphenyl, 2,6-Dichlorphenyl, 2-Chlor-6-fluorphenyl.

10. Verbindungen der Formel (IIa) gemäß Anspruch 8, in welcher
Ar¹ für einfach oder zweifach, gleich oder verschieden substituiertes Phenyl steht, wobei als Substituenten genannt seien:
Fluor, Chlor, Brom, C₂-C₄-Alkoxy, durch Fluor und/oder Chlor substituiertes C₁-C₄-Alkoxy, gegebenenfalls einfach oder mehrfach durch Fluor und/oder Chlor substituiertes C₃-C₆-Cycloalkyloxy und einfach oder zweifach, gleich oder verschieden durch Fluor, Chlor, Brom, C₁-C₄-Alkyl, gegebenenfalls durch Fluor und/oder Chlor substituiertes C₁-C₄-Alkoxy, oder gegebenenfalls einfach oder mehrfach durch Fluor und/oder Chlor substituiertes C₃-C₆-Cycloalkyloxy substituiertes Phenyl,
ausgenommen die Verbindung 1-(5-Brom-2-thienyl)-2-hydroxyethanonoxim) und ausgenommen Verbindungen, in welcher Ar¹ eine der folgenden Bedeutungen annimmt:
4-Chlorphenyl, 2-Chlorphenyl, 2-Bromphenyl, 2,6-Dichlorphenyl, 2-Chlor-6-fluorphenyl.

## Claims

1. Process for the preparation of compounds of the formula (I) 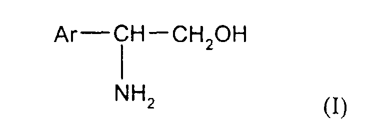 in which
Ar represents in each case identically or differently monosubstituted to pentasubstituted phenyl or in each case identically or differently monosubstituted to trisubstituted thienyl, pyridyl or pyrimidinyl, where as substituents there may be mentioned in each case:
fluorine, chlorine, bromine, C₁-C₆-alkyl, C₁-C₆-halogenoalkyl, C₁-C₆-alkoxy, C₁-C₆-halogenoalkoxy, C₃-C₆-cycloalkyloxy unsubstituted or substituted by halogen or C₁-C₆-alkyl, and phenyl unsubstituted or identically or differently monosubstituted to pentasubstituted by fluorine, chlorine, bromine, C₁-C₆-alkyl, C₁-C₆-halogenoalkyl, C₁-C₆-alkoxy, C₁-C₆-halogenoalkoxy or C₃-C₆-cycloalkyloxy unsubstituted or substituted by halogen or C₁-C₆-alkyl,
characterized in that compounds of the formula (II) 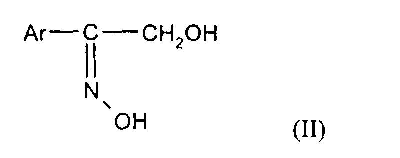 in which
Ar has the meaning given above,
are catalytically hydrogenated using Raney nickel or Raney cobalt in the presence of a diluent and in the presence of a base.

2. Process for the preparation of compounds of the formula (I) according to Claim 1, in which
Ar represents in each case identically or differently monosubstituted to trisubstituted phenyl, thienyl, pyridyl or pyrimidinyl, where as substituents there may be mentioned:
fluorine, chlorine, bromine, C₁-C₄-alkyl, C₁-C₄-halogenoalkyl, C₁-C₄-alkoxy, C₁-C₄-halogenoalkoxy, C₃-C₆-cycloalkyloxy unsubstituted or substituted by halogen, and phenyl unsubstituted or identically or differently monosubstituted to trisubstituted by fluorine, chlorine, bromine, C₁-C₄-alkyl, C₁-C₄-halogenoalkyl, C₁-C₄-alkoxy, C₁-C₄-halogenoalkoxy or C₃-C₆-cycloalkyloxy unsubstituted or substituted by halogen.

3. Process for the preparation of compounds of the formula (1) according to Claim 1, in which
Ar represents identically or differently monosubstituted or disubstituted phenyl, where as substituents there may be mentioned: fluorine, chlorine, bromine, C₁-C₄-alkyl unsubstituted or substituted by fluorine and/or chlorine, C₁-C₄-alkoxy unsubstituted or substituted by fluorine and/or chlorine, C₃-C₆-cycloalkyloxy unsubstituted or monosubstituted or polysubstituted by fluorine and/or chlorine and phenyl unsubstituted or identically or differently monosubstituted or disubstituted by fluorine, chlorine, bromine, C₁-C₄-alkyl unsubstituted or substituted by fluorine and/or chlorine, C₁-C₄-alkoxy unsubstituted or substituted by fluorine and/or chlorine or C₃-C₆-cycloalkyloxy unsubstituted or monosubstituted or polysubstituted by fluorine and/or chlorine.

4. Compounds of the formula (Ia) 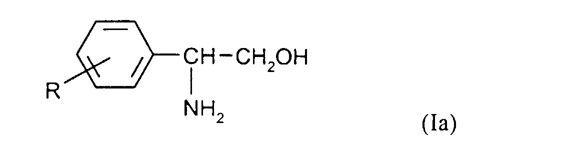 in which
R represents a radical 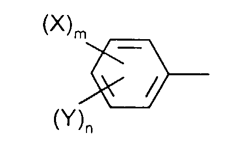
in which
X represents halogen, C₁-C₆-alkyl or C₁-C₆-alkoxy,
Y represents C₁-C₆-halogenoalkoxy or halogen-substituted C₃-C₆-cycloalkyloxy,
m represents the number 0, 1 or 2 and
n represents the number 1 or 2.

5. Compounds of the formula (Ia) according to Claim 4, in which
X represents fluorine, chlorine, bromine, C₁-C₄-alkyl or C₁-C₄-alkoxy,
Y represents C₁-C₄-halogenoalkoxy or halogen-substituted C₃-C₆-cycloalkoxy,
n represents the number 1 and
m represents 0 or 1.

6. Compounds of the formula (Ia) according to Claim 4, in which
R represents a radical 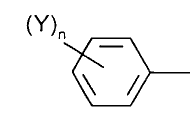
in which
Y represents fluorine- and/or chlorine-substituted C₁-C₄-alkoxy or fluorine- and/or chlorine-substituted C₃-C₆-cycloalkoxy and
n represents the number 1.

7. Compounds of the formula (Ia) according to claim 4, in which
R represents a radical 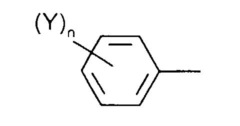
in which
Y represents one of the following radicals:
-OCHF₂, -OCClF₂, -OCF₂CHFCl, -OCF₂CH₂F, -OCF₂CHF₂, -OCF₂CCl₃, -OCF₂CHFCF₃, -OCH₂CF₃, -OCH₂CF₂CHF₂, -OCH₂CF₂CF₃, 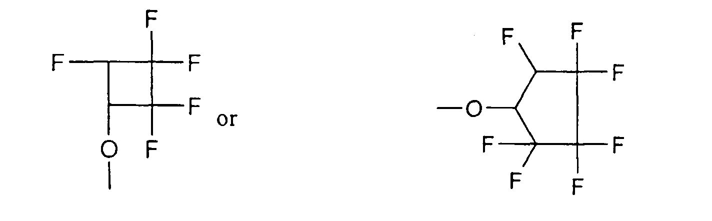 and
n represents the number 1.

8. Compounds of the formula (IIa) 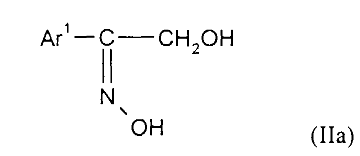 in which
Ar¹ represents identically or differently monosubstituted to pentasubstituted phenyl or in each case identically or differently monosubstituted to trisubstituted thienyl, pyridyl or pyrimidinyl, where as substituents there may be mentioned:
fluorine, chlorine, bromine, C₂-C₆-alkoxy, C₁-C₆-halogenoalkoxy, C₃-C₆-cycloalkyloxy unsubstituted or substituted by halogen or C₁-C₆-alkyl, and phenyl unsubstituted or identically or differently monosubstituted to pentasubstituted by fluorine, chlorine, bromine, C₁-C₆-alkyl, C₁-C₆-alkoxy, C₁-C₆-halogenoalkoxy or C₃-C₆-cycloalkyloxy unsubstituted or substituted by halogen or C₁-C₆-alkyl,
except for the compound 1-(5-bromo-2-thienyl)-2-hydroxy-ethanone oxime and except for compounds in which Ar¹ is one of the following:
4-chlorophenyl, 2-chlorophenyl, 2-bromophenyl, 2,6-dichlorophenyl, 2-chloro-6-fluorophenyl or pentafluorophenyl.

9. Compounds of the formula (IIa) according to Claim 8, in which
Ar¹ represents in each case identically or differently monosubstituted to trisubstituted phenyl, thienyl, pyridyl or pyrimidinyl, where as substituents there may be mentioned:
fluorine, chlorine, bromine, C₂-C₄-alkoxy, C₁-C₄-halogenoalkoxy, C₃-C₆-cycloalkyloxy unsubstituted or substituted by halogen and phenyl identically or differently monosubstituted to trisubstituted by fluorine, chlorine, bromine, C₁-C₄-alkyl, C₁-C₄-alkoxy, C₁-C₄-halogenoalkoxy or C₃-C₆-cycloalkyloxy unsubstituted or substituted by halogen,
except for the compound 1-(5-bromo-2-thienyl)-2-hydroxy-ethanone oxime and except for compounds in which Ar¹ is one of the following:
4-chlorophenyl, 2-chlorophenyl, 2-bromophenyl, 2,6-dichlorophenyl or 2-chloro-6-fluorophenyl.

10. Compounds of the formula (IIa) according to Claim 8, in which
Ar¹ represents identically or differently monosubstituted or disubstituted phenyl, where as substituents there may be mentioned:
fluorine, chlorine, bromine, C₂-C₄-alkoxy, C₁-C₄-alkoxy substituted by fluorine and/or chlorine, C₃-C₆-cycloalkyloxy unsubstituted or monosubstituted or polysubstituted by fluorine and/or chlorine, and phenyl identically or differently monosubstituted or disubstituted by fluorine, chlorine, bromine, C₁-C₄-alkyl, C₁-C₄-alkoxy unsubstituted or substituted by fluorine and/or chlorine or C₃-C₆-cycloalkyloxy unsubstituted or monosubstituted or polysubstituted by fluorine and/or chlorine,
except for the compound 1-(5-bromo-2-thienyl)-2-hydroxy-ethanone oxime and except for compounds in which Ar¹ is one of the following:
4-chlorophenyl, 2-chlorophenyl, 2-bromophenyl, 2,6-dichlorophenyl or 2-chloro-6-fluorophenyl.

## Revendications

1. Procédé de production de composés de formule (I) 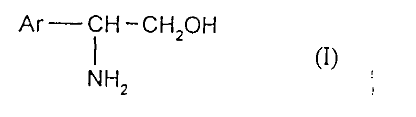 dans laquelle
Ar est un groupe phényle portant, dans chaque cas, un à cinq substituants identiques ou différents ou un groupe thiényle, pyridyle ou pyrimidinyle dont chacun est substitué une à trois fois identiques ou différentes, avec comme substituants dans chaque cas :
fluor, chlore, brome, alkyle en C₁ à C₆, halogénalkyle en C₁ à C₆, alkoxy en C₁ à C₆, halogénalkoxy en C₁ à C₆, cycloalkyloxy en C₃ à C₆ éventuellement substitué par un halogène ou un radical alkyle en C₁ à C₆ et phényle substitué, le cas échéant, une à cinq fois identiques ou différentes par du fluor, du chlore, du brome, un radical alkyle en C₁ à C₆, halogénalkyle en C₁ à C₆, alkoxy en C₁ à C₆, halogénalkoxy en C₁ à C₆ ou cycloalkyloxy en C₃ à C₆ éventuellement substitué par un halogène ou un radical alkyle en C₁ à C₆,
caractérisé en ce qu'on effectue l'hydrogénation catalytique de composés de formule (II) 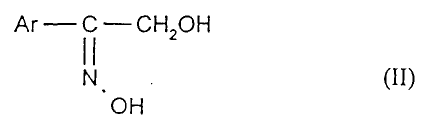 dans laquelle
Ar a la définition indiquée ci-dessus,
avec du nickel de Raney ou du cobalt de Raney en présence d'un diluant et en présence d'une base.

2. Procédé de production de composés de formule (I) suivant la revendication 1, dans laquelle
Ar représente un groupe phényle, thiényle, pyridyle ou pyrimidinyle portant chacun un à trois substituants identiques ou différents, avec comme substituants :
le fluor, le chlore, le brome, un radical alkyle en C₁ à C₄, halogénalkyle en C₁ à C₄, alkoxy en C₁ à C₄, halogénalkoxy en C₁ à C₄, cycloalkyloxy en C₃ à C₆ éventuellemenet substitué par un halogène et phényle éventuellement substitué une à trois fois identiques ou différentes par du fluor, du chlore, du brome, un radical alkyle en C₁ à C₄, halogénalkyle en C₁ à C₄, alkoxy en C₁ à C₄, halogénalkoxy en C₁ à C₄ ou cycloalkyloxy en C₃ à C₆ éventuellement substitué par un halogène.

3. Procédé de production de composés de formule (I) suivant la revendication 1, dans laquelle
Ar est un groupe phényle substitué une ou deux fois identiques ou différentes, avec comme substituants : le fluor, le chlore, le brome, un groupe alkyle en C₁ à C₄ éventuellement substitué par du fluor et/ou du chlore, un groupe alkoxy en C₁ à C₄ éventuellement substitué par du fluor et/ou du chlore, un groupe cycloalkyloxy en C₃ à C₆ éventuellement substitué une ou plusieurs fois par du fluor et/ou du chlore et un groupe phényle éventuellement substitué une ou deux fois identiques ou différentes par du fluor, du chlore, du brome, un radical alkyle en C₁ à C₄ éventuellement substitué par du fluor et/ou du chlore, un radical alkoxy en C₁ à C₄ éventuellement substitué par du fluor et/ou du chlore ou un radical cycloalkyloxy en C₃ à C₆ éventuellement substitué une ou plusieurs fois par du fluor et/ou du chlore.

4. Composés de formule (Ia) 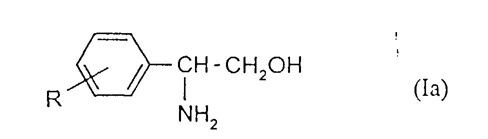 dans laquelle
R représente un reste 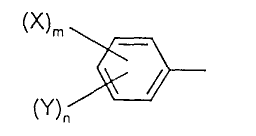
dans lequel
X représente un halogène, un groupe alkyle en C₁ à C₆ ou alkoxy en C₁ à C₆,
Y est un groupe halogénalkoxy en C₁ à C₆ ou un groupe cycloalkyloxy en C₃ à C₆ substitué par un halogène,
m représente le nombre 0, 1 ou 2 et
n représente le nombre 1 ou 2.

5. Composés de formule (Ia) suivant la revendication 4, dans laquelle
X représente le fluor, le chlore, le brome, un groupe alkyle en C₁ à C₄ ou alkoxy en C₁ à C₄,
Y est un groupe halogénalkoxy en C₁ à C₄ ou un groupe cycloalkoxy en C₃ à C₆ substitué par un halogène,
n représente le nombre 1 et
m a la valeur 0 ou 1.

6. Composés de formule (Ia) suivant la revendication 4, dans laquelle
R représente un reste 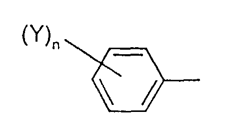
dans lequel
Y est un groupe alkoxy en C₁ à C₄ substitué par du fluor et/ou du chlore ou un groupe cycloalkoxy en C₃ à C₆ substitué par du fluor et/ou du chlore et
n représente le nombre 1.

7. Composés de formule (Ia) suivant la revendication 4, dans laquelle
R est un reste 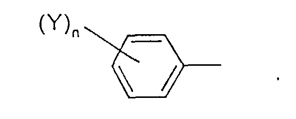
dans lequel
Y représente l'un des restes suivants :
-OCHF₂, -OCClF₂, -OCF₂CHFCl, -OCF₂CH₂F, -OCF₂CHF₂, -OCF₂CCl₃, -OCF₂CHFCF₃, -OCH₂CF₃, -OCH₂CF₂CHF₂, -OCH₂CF₂CF₃, 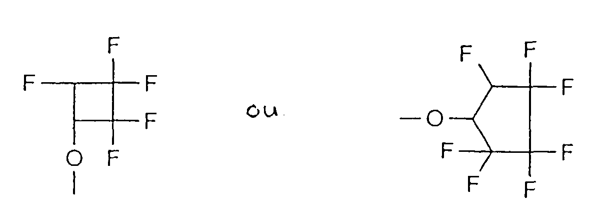 et
n représente le nombre 1.

8. Composés de formule (IIa) 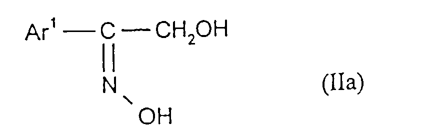 dans laquelle
Ar¹ est un groupe phényle substitué une à cinq fois identiques ou différentes ou un groupe thiényle, pyridyle ou pyrimidinyle dont chacun est substitué une à trois fois identiques ou différentes, avec comme substituants :
le fluor, le chlore, le brome, un groupe alkoxy en C₂ à C₆, halogénalkoxy en C₁ à C₆, un groupe cycloalkyloxy en C₃ à C₆ éventuellement substitué par un halogène ou un radical alkyle en C₁ à C₆ et un groupe phényle substitué une à cinq fois identiques ou différentes par du fluor, du chlore, du brome, un radical alkyle en C₁ à C₆, alkoxy en C₁ à C₆, halogénalkoxy en C₁ à C₆ ou cycloalkyloxy en C₃ à C₆ portant éventuellement un substituant halogéno ou alkyle en C₁ à C₆,
excepté le composé 1-(5-bromo-2-thiényl)-2-hydroxy-éthanonoxime et excepté les composés dans lesquels Ar¹ reçoit l'une des définitions suivantes :
4-chlorophényle, 2-chlorophényle, 2-bromophényle, 2,6-dichlorophényle, 2-chloro-6-fluorophényle, pentafluorophényle.

9. Composés de formule (IIa) suivant la revendication 8, dans laquelle
Ar¹ est un groupe phényle, thiényle, pyridyle ou pyrimidinyle, chacun portant un à trois substituants identiques ou différents, avec comme substituants :
le fluor, le chlore, le brome, un groupe alkoxy en C₂ à C₄, halogénalkoxy en C₁ à C₄, un groupe cycloalkyloxy en C₃ à C₆ éventuellement substitué par un halogène et un groupe phényle substitué une à trois fois identiques ou différentes par du fluor, du chlore, du brome, un radical alkyle en C₁ à C₄, alkoxy en C₁ à C₄, halogénalkoxy en C₁ à C₄ ou un radical cycloalkyloxy en C₃ à C₆ éventuellement substitué par un halogène, excepté le composé 1-(5-bromo-2-thiényl)-2-hydroxy-éthanonoxime et excepté les composés dans lesquels Ar¹ reçoit l'une des définitions suivantes :
4-chlorophényle, 2-chlorophényle, 2-bromophényle, 2,6-dichlorophényle et 2-chloro-6-fluorophényle.

10. Composés de formule (IIa) suivant la revendication 8, dans laquelle
Ar¹ est un groupe phényle substitué une ou deux fois identiques ou différentes, avec comme substituants :
le fluor, le chlore, le brome, un groupe alkoxy en C₂ à C₄, un groupe alkoxy en C₁ à C₄ éventuellement substitué par du fluor et/ou du chlore, un groupe cycloalkyloxy en C₃ à C₆ éventuellement substitué une ou plusieurs fois par du fluor et/ou du chlore et un groupe phényle substitué une ou deux fois identiques ou différentes par du fluor, du chlore, du brome, un radical alkyle en C₁ à C₄, un radical alkoxy en C₁ à C₄ éventuellement substitué par du fluor et/ou du chlore ou un radical cycloalkyloxy en C₃ à C₆ éventuellement substitué une ou plusieurs fois par du fluor et/ou du chlore,
excepté le composé 1-(5-bromo-2-thiényl)-2-hydroxy-éthanonoxime et excepté les composés dans lesquels Ar¹ reçoit l'une des définitions suivantes :
4-chlorophényle, 2-chlorophényle, 2-bromophényle, 2,6-dichlorophényle, 2-chloro-6-fluorophényle.m
